# EUROPEAN PATENT APPLICATION

(11) **EP 3 613 434 A1**
(43) Date of publication of application: **26.02.2020**
(21) Application number: 18788650.2
(22) Date of filing: 12.04.2018
(51) Int. Cl.: A61K 41/00, A61K 31/131, A61K 31/197, A61K 31/221, A61K 31/407, A61K 31/4985, A61K 49/00, A61P 35/00, A61P 43/00

(54) **ENHANCER OF PHOTODYNAMIC EFFECT IN ALA-PDT OR ALA-PDD**

(30) Priority: 18.04.2017 JP 2017082167
(71) Applicant: SBI Pharmaceuticals Co., Ltd., Tokyo 106-6020 (JP)
(72) Inventor: ISHIZUKA, Masahiro, Tokyo 106-6020 (JP); ISHII, Takuya, Kobe-Shi Hyogo 650-0047 (JP); NAKAJIMA, Motowo, Tokyo 106-6020 (JP); KITAJIMA, Yuya, Kobe-Shi Hyogo 650-0047 (JP)
(74) Representative: Weickmann & Weickmann PartmbB
(86) International application number: PCT/JP2018/015363
(87) International publication number: WO 2018/193958

(57) **Abstract**

The object of the present invention is to potentiate the photodynamic effect in ALA-PDT and ALA-PDD. The present invention provides a pharmaceutical related to a combination of ALAs and a dynamin inhibitor. [Selected Drawings] None

## Description

### Technical Field

The present invention relates to a photodynamic effect potentiator for photodynamic therapy (abbreviated PDT) or photodynamic diagnosis (PDD).

### Background Art

Photodynamic therapy (PDT) is cited as one choice of cancer therapy that is different from anticancer agent therapy or radiation therapy. Cancer therapy by PDT is a therapeutic method of treating cancer by administering a light-sensitive substance (photosensitizer) that has the nature to accumulate in cancer cells to a subject and irradiating light of a particular wavelength to the cancer tissue, and has been gathering attention in recent years owing to the fact that it is a therapeutic method that is minimally invasive and has less chance of leaving a therapeutic scar.

Moreover, in recent years, photodynamic diagnosis (PDD) has been gathering attention as a method for diagnosing diseased tissue such as cancer, wart, and acne. PDD is a diagnostic method for identifying the target site by administering to a subject a light-sensitive substance (photosensitizer) that has the nature to accumulate in target tissue and irradiating light of a particular wavelength. It has the advantage compared to previous diagnostic methods in that it has less burden on patients, owing to the fact that it is minimally invasive and has less side effects.

As described above, in PDT and PDD, a compound that reacts to light (photosensitizer) that has the nature to accumulate in the tissue to be the target of therapy or detection is also utilized. Various substances are being investigated as photosensitizers that are favorably employed for PDT and PDD. In recent years, "ALA-PDT" or "ALA-PDD" that combines PDT or PDD with administration to a subject of 5-aminolevulinic acids (also referred to herein as "ALAs") which are precursors of porphyrin as well as biological substances has been gathering attention (e.g. Patent Literature 1).

Although ALAs do not have light sensitivity per se, ALAs are metabolically activated in cells by a group of series of enzymes in the heme biosynthetic pathway to become porphyrin (mainly protoporphyrin IX(PpIX)). Porphyrins have the nature to be incorporated into tumor cells and accumulated, and since PpIX is a photosensitizer that has peaks at 410 nm, 510 nm, 545 nm, 580 nm, 630 nm, etc., they can be utilized for PDT or PDD. Since ALA is metabolized *in vivo* and excreted within 48 hours, it has the characteristic of having almost no influence on the general light sensitivity of the body.

However, it has been confirmed that depending on the cancer cell type or the degree of malignancy, there are some cells where one member of the ABC transporter family, ABCG2, of which the substrate is porphyrins such as PpIX, is highly expressed, thus causing PpIX to be exported into the cytoplasm, and PpIX is not sufficiently accumulated (e.g. Non-Patent Literature 1).

### Citation List

[Patent Literature 1] Japanese Published Unexamined Patent Application Publication No. Hei11-12197

[Non-Patent Literature 1] Drug Metab. Pharmacokinet. 22(6):428-440(2007)

### Summary of the Invention

### Problems to be Solved by the Invention

Until now, methods of combined use of ABCG2 inhibitors or methods of combined use of anticancer agents which are ABCG2 transporter substrates have been investigated in order to potentiate the photodynamic effect in ALA-PDT and ALA-PDD. However, high expression of ABCG2 is not necessarily confirmed in all cancer cells, and there are some cancer cells where adversely the expression of ABCG2 is low, and it is thought that there is a limit to potentiating the accumulation of PpIX in cells by merely inhibition of ABCG2.

### Means for Solving the Problems

As a result of extensive investigation by the present inventors for substances that increase the amount of PpIX accumulated in cells, it was found that by using a dynamin inhibitor in combination with ALAs, the amount of PpIX accumulated is increased in almost all cancer cells compared to when ALAs were applied alone. Further, the present inventors found, in particular, that an inhibitor that targets the pleckstrin homology domain of dynamin is useful in increasing the amount of PpIX accumulated in cells when ALAs are applied.

In other words, in one embodiment, the present invention relates to a pharmaceutical for potentiating photodynamic reaction in photodynamic therapy or photodynamic diagnosis, characterized in that it comprises:
(A) a compound shown by the following Formula (I): 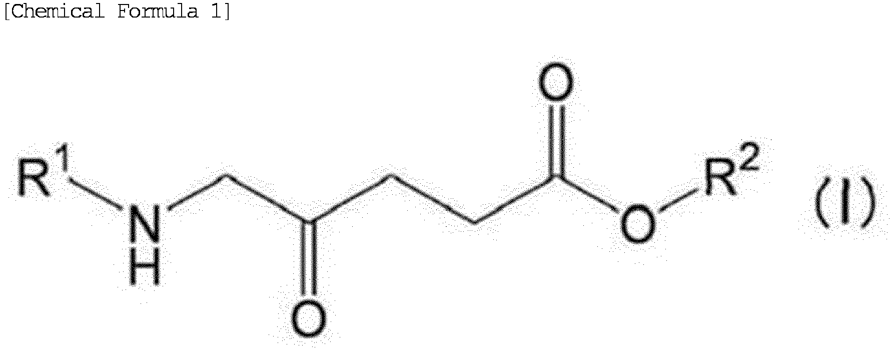
   (in which R¹ represents a hydrogen atom or an acyl group, and R² represents a hydrogen atom, a linear or branched alkyl group, a cycloalkyl group, an aryl group, or an aralkyl group)
   or a salt thereof, and
(B) a dynamin inhibitor.

In one embodiment, the present invention also relates to a combination pharmaceutical which is administered at same or different times for potentiating photodynamic reaction in photodynamic therapy or photodynamic diagnosis, which is;
(A) a compound shown by the following Formula (I): 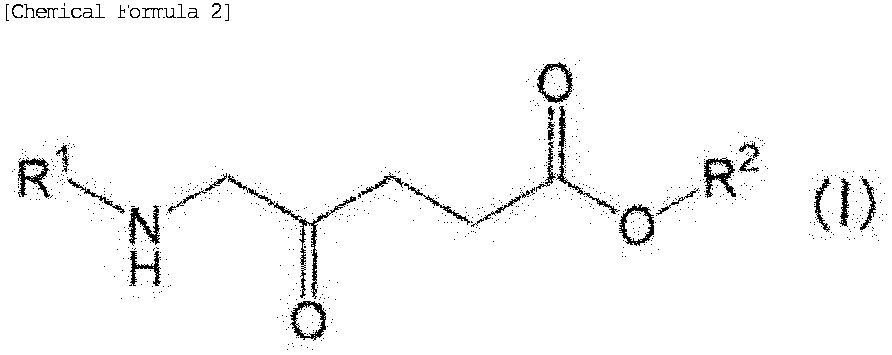
   (in which R¹ represents a hydrogen atom or an acyl group, and R² represents a hydrogen atom, a linear or branched alkyl group, a cycloalkyl group, an aryl group, or an aralkyl group)
   or a salt thereof, and
(B) a dynamin inhibitor.

In one embodiment, the present invention also relates to a pharmaceutical for potentiating photodynamic reaction in photodynamic therapy or photodynamic diagnosis, characterized in that said pharmaceutical comprises a compound shown by the following Formula (I): 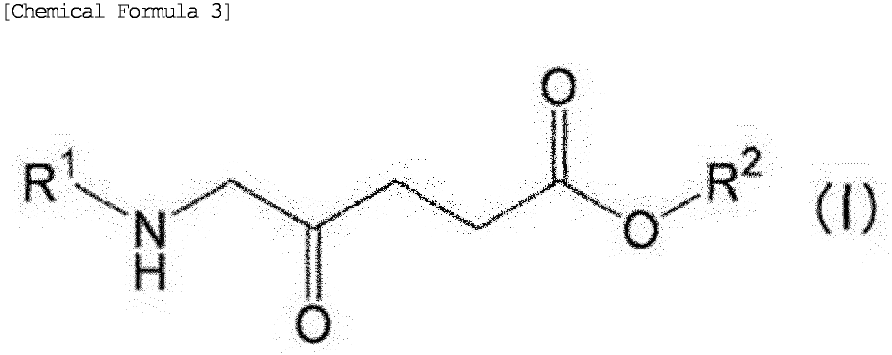
(in which R¹ represents a hydrogen atom or an acyl group, and R² represents a hydrogen atom, a linear or branched alkyl group, a cycloalkyl group, an aryl group, or an aralkyl group)
or a salt thereof, and
   said pharmaceutical is used in combination with a dynamin inhibitor.

Moreover, one embodiment of the present invention is characterized in that in the above pharmaceutical, R¹ is selected from the group consisting of a hydrogen atom, an alkanoyl group having 1 - 8 carbons, and an aroyl group having 7 - 14 carbons, and R² is selected from the group consisting of a hydrogen atom, a linear or branched alkyl group having 1 - 8 carbons, a cycloalkyl group having 3 - 8 carbons, an aryl group having 6 - 14 carbons, and an aralkyl group having 7 - 15 carbons.

Moreover, one embodiment of the present invention is characterized in that in the above pharmaceutical, R¹ and R² are hydrogen atoms.

Moreover, one embodiment of the present invention is characterized in that in the above pharmaceutical, said dynamin inhibitor is a dynamin inhibitor that targets the pleckstrin homology domain of dynamin.

Moreover, one embodiment of the present invention is characterized in that in the above pharmaceutical, said dynamin inhibitor that targets the pleckstrin homology domain of dynamin is one or more compound selected from the group consisting of the following Formula (II) - Formula (VI).

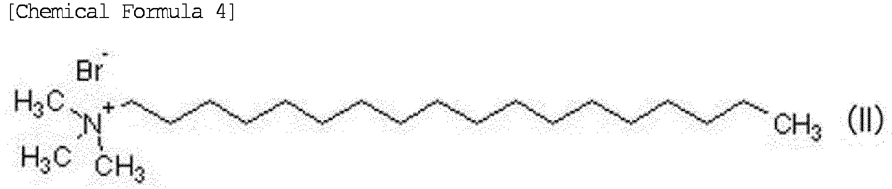

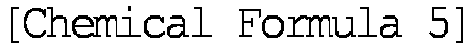

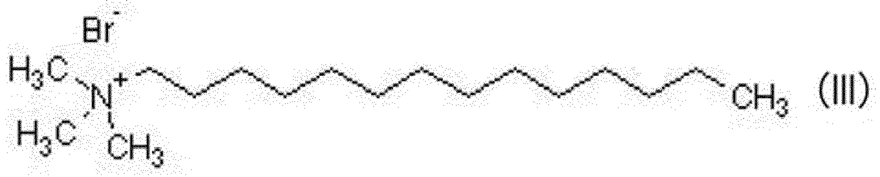

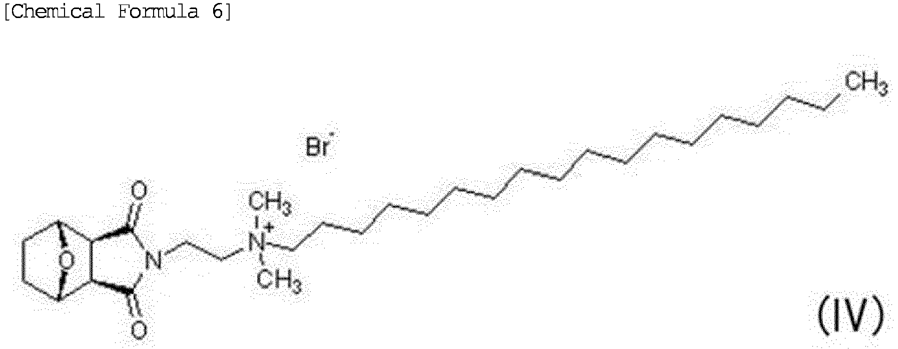

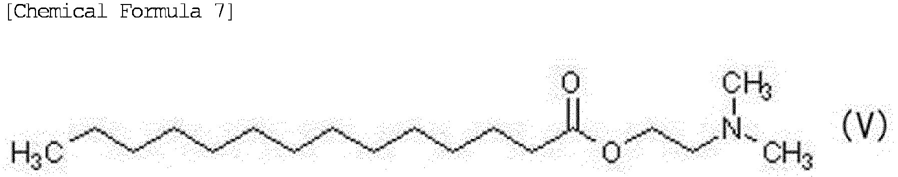

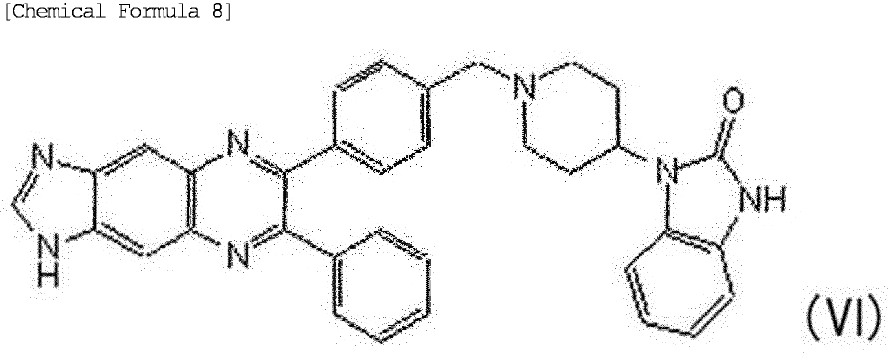

Moreover, one embodiment of the present invention is characterized in that in the above combination pharmaceutical, said (A) and said (B) are prepared as a kit.

Another embodiment of the present invention relates to a method for potentiating photodynamic reaction in photodynamic therapy or photodynamic diagnosis that employs a compound shown by the following Formula (I): 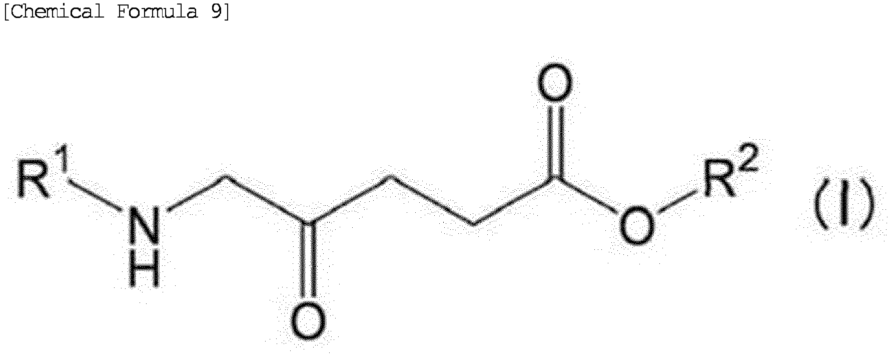
(in which R¹ represents a hydrogen atom or an acyl group, and R² represents a hydrogen atom, a linear or branched alkyl group, a cycloalkyl group, an aryl group, or an aralkyl group)
or a salt thereof, characterized in that it comprises:
   a step of administering to a subject a therapeutically effective amount of a dynamin inhibitor.

Note that an invention of any combination of one or more characteristics of the present invention described above is also encompassed by the scope of the present invention.

### Brief Description of the Drawings

Figure 1 is graphs showing the effect of increasing the amount of PpIX accumulated in cells when using dynamin inhibitors (OcTMAB, Iminodyn22) in combination with ALA.
Figure 2 is graphs showing the effect of increasing the amount of PpIX accumulated in cells when using dynamin inhibitors (OcTMAB) and FTC in combination with ALA.
Figure 3 is graphs showing the effect of increasing the amount of PpIX accumulated in cells when using a dynamin inhibitor (MiTMAB) in combination with ALA.
Figure 4 is graphs showing the effect of increasing the amount of PpIX accumulated in cells when using a dynamin inhibitor (RTIL13) in combination with ALA.
Figure 5 is graphs showing the effect of increasing the amount of PpIX accumulated in cells when using an AKT inhibitor VIII in combination with ALA.

### Description of Embodiments

ALAs as used herein refers to refers to an ALA or a derivative thereof, or salts thereof.

ALA as used herein means 5-aminolevulinic acid. ALA is also referred to as δ-aminolevulinic acid, and is a type of amino acid.

The compound shown by the following Formula (I) can be exemplified as an example of an ALA derivative. In Formula (I), R¹ represents a hydrogen atom or an acyl group, and R² represents a hydrogen atom, a linear or branched alkyl group, a cycloalkyl group, an aryl group, or an aralkyl group. Note that in Formula (I), ALA corresponds to when R¹ and R² are hydrogen atoms.

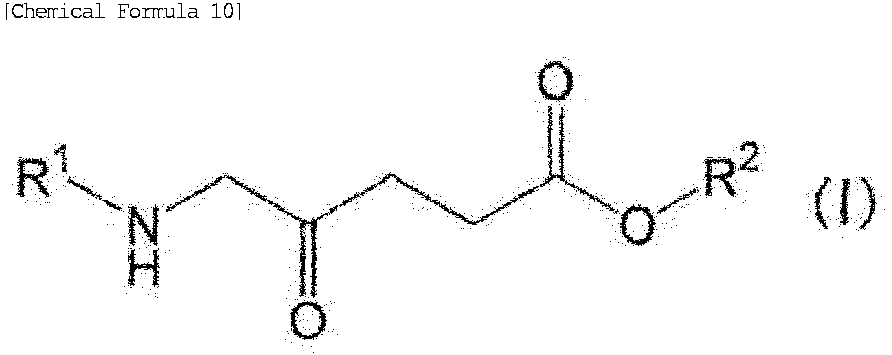

ALAs may act as an active ingredient *in vivo* in the form of the ALA of Formula (I) or a derivative thereof, and can also be administered as a prodrug (precursor) that is degradated by an *in vivo* enzyme.

The acyl group in R¹ of Formula (I) can include a linear or branched alkanoyl group having 1 - 8 carbons such as formyl, acetyl, propionyl, butyryl, isobutyryl, valeryl, isovaleryl, pivaloyl, hexanoyl, octanoyl, and benzylcarbonyl groups, or an aroyl group having 7 - 14 carbons such as benzoyl, 1-naphthoyl, and 2-naphthoyl groups.

The alkyl group in R² of Formula (I) can include a linear or branched alkyl group having 1 - 8 carbons such as methyl, ethyl, propyl, isopropyl, butyl, isobutyl, sec-butyl, tert-butyl, pentyl, isopentyl, neopentyl, hexyl, heptyl, and octyl groups.

The cycloalkyl group in R² of Formula (I) can include a cycloalkyl group having 3 - 8 carbons which may be saturated or have partially unsaturated bonds, such as cyclopropyl, cyclobutyl, cyclopentyl, cyclohexyl, cycloheptyl, cyclooctyl, cyclododecyl, and 1-cyclohexenyl groups.

The aryl group in R² of Formula (I) can include an aryl group having 6 - 14 carbons such as phenyl, naphthyl, anthryl, and phenanthryl groups.

The aralkyl group in R² of Formula (I) can be exemplified with the same aryl groups as above as the aryl moiety and the same alkyl groups as above as the alkyl moiety, and can specifically include an aralkyl group having 7 - 15 carbons such as benzyl, phenethyl, phenylpropyl, phenylbutyl, benzhydryl, trityl, naphthylmethyl, and naphthylethyl groups.

Preferred ALA derivatives include compounds where R¹ is a formyl group, an acetyl group, a propionyl group, a butyryl group, and the like. Moreover, preferred ALA derivatives also include compounds where the above R² is a methyl group, an ethyl group, a propyl group, a butyl group, a pentyl group, and the like. Moreover, preferred ALA derivatives also include compounds where the combination of the above R¹ and R² is each combination of (formyl and methyl), (acetyl and methyl), (propionyl and methyl), (butyryl and methyl), (formyl and ethyl), (acetyl and ethyl), (propionyl and ethyl), and (butyryl and ethyl).

Among ALAs, a salt of an ALA or a derivative thereof can include a pharmaceutically acceptable acid addition salt, a metal salt, an ammonium salt, an organic amine addition salt, and the like. Acid addition salts can be exemplified by e.g. each of inorganic acid salts such as a hydrochloride salt, a hydrobromide salt, a hydroiodide salt, a phosphate salt, a nitrate salt, and a sulfate salt, and each of organic acid addition salts such as a formate salt, an acetate salt, a propionate salt, a toluenesulfate salt, a succinate salt, an oxalate salt, a lactate salt, a tartrate salt, a glycolate salt, a methanesulfonate salt, a butyrate salt, a valerate salt, a citrate salt, a fumarate salt, a maleate salt, and a malate salt. Metal salts can be exemplified by each of alkali metal salts such as a lithium salt, a sodium salt, and a potassium salt, each of alkaline earth metal salts such as a magnesium salt and a calcium salt, and each of metal salts such as aluminum and zinc. Ammonium salts can be exemplified by e.g. ammonium salts and alkyl ammonium salts such as a tetramethylammonium salt. Organic amine salts can be exemplified by each of salts such as a triethylamine salt, a piperidine salt, a morpholine salt, and a toluidine salt. Note that these salts can also be employed as a solution at the time of use.

Among the above ALAs, the most favorable are ALA and various esters such as an ALA methyl ester, an ALA ethyl ester, an ALA propyl ester, an ALA butyl ester, and an ALA pentyl ester, as well as hydrochloride salts, phosphate salts, and sulfate salts thereof. In particular, ALA hydrochloride salts and ALA phosphate salts can be exemplified as particularly favorable.

The above ALAs can be manufactured by e.g. well-known methods such as chemical synthesis, production by microorganisms, and production by enzymes. Moreover, the above ALAs may also form a hydrate or a solvate, and ALAs can be employed alone or in an appropriate combination of two or more.

When the above ALAs are to be prepared as an aqueous solution, attention must be paid so that the aqueous solution will not become alkaline in order to prevent degradation of ALAs. In the case it becomes alkaline, degradation can be prevented by removing oxygen.

ALA-PDT as used herein means photodynamic therapy (PDT) that employs ALAs, most typically PDT that employs ALA. Moreover, ALA-PDD means photodynamic diagnosis (PDD) that employs ALAs, most typically PDD that employs ALA.

In the above ALA-PDT, when performing PDT that treats the target site by administering to a subject a light-sensitive substance (photosensitizer) that has the nature to accumulate in target tissue and irradiating a ray of light of a particular wavelength, ALAs which do not have a photosensitization effect per se is administered to a subject. In the body of the subject, porphyrin (mainly PpIX) induced from ALAs via the pigment biosynthetic pathway is allowed to accumulate at the target site, and the accumulated PpIX is excited to allow photoexcitation of the surrounding oxygen molecules. The singlet oxygens produced as a result have a cytotoxic effect due to their intense oxidative power. For ALA-PDT, it is preferred to employ e.g. a ray of light having peak at 400 nm - 420 nm or a ray of light having peak at 600 nm - 650 nm.

On the other hand, in the above ALA-PDD, when performing PDD that treats the target site by administering to a subject a light-sensitive substance (photosensitizer) that has the nature to accumulate in target tissue and irradiating a ray of light, ALAs which do not have a photosensitization effect per se is administered to a subject. In the body of the subject, porphyrin (mainly PpIX) induced from ALAs via the pigment biosynthetic pathway is allowed to accumulate at the target site, the accumulated PpIX is excited, and the fluorescence is detected to detect or diagnose the presence or absence of the target tissue. For ALA-PDD, it is preferred to employ e.g. a ray of light having peak at 400 nm - 420 nm.

Dynamin is a high molecular weight GTPase, and it is known to be associated with membrane formation or shape alteration in various organelles. Dynamin is also known to be a protein comprising a GTPase domain (GTPase Allosteric Site domain: GAS domain), BSE (bundle signaling element), a handle-like structure (stalk), and a pleckstrin homology domain (PH domain).

The pleckstrin homology domain is a module consisting of about 120 amino acid residues, and is known to be found not only in dynamin but also in a variety of signal transduction proteins involved in intracellular kinetics, cellular signal transduction, and cellular skeleton reconstruction.

The dynamin inhibitor employed herein is not particularly limited as long as it is a substance that inhibits the function of dynamin. In the present invention, among dynamin inhibitors, in particular, an inhibitor that targets the pleckstrin homology domain (PH domain) of dynamin is favorably employed.

An "inhibitor that targets the PH domain of dynamin" as used herein may be an agent generally known as an inhibitor that targets the PH domain of dynamin (such as OcTMAB™ (from abcam), MiTMAB™ (from abcam), RTIL13™ (from abcam), and Pro-myristic acid (from abcam)), but is not limited thereto, and any substance that is known to bind to the PH domain may be employed as the "inhibitor that targets the PH domain of dynamin" of the present invention. For example, an inhibitor of Akt that possesses a pH domain in the molecule (such as Akt Inhibitor VIII (from Merck Millipore)) is also included in "an inhibitor that targets the PH domain of dynamin" of the present invention.

OcTMAB™ that may be employed in the present invention is a compound shown by the following Formula (II).

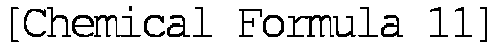

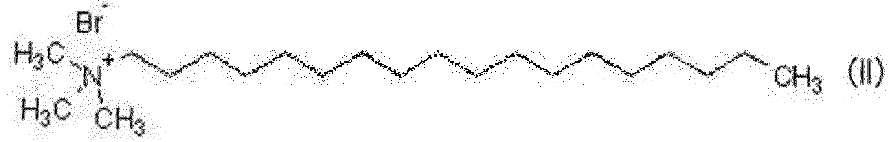

MiTMAB™ that may be employed in the present invention is a compound shown by the following Formula (III).

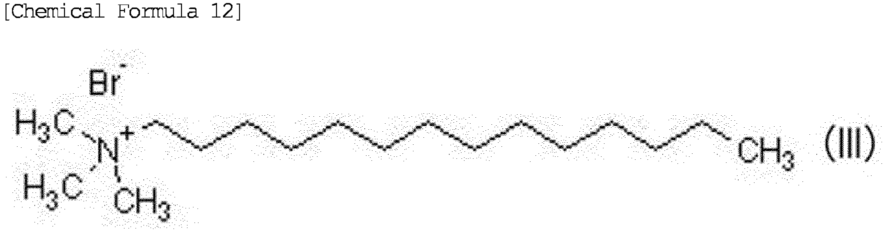

RTIL13™ that may be employed in the present invention is a compound shown by the following Formula (IV).

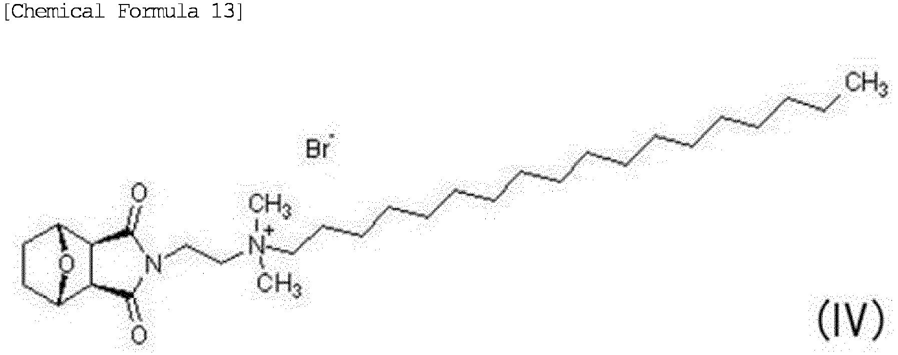

Pro-myristic acid that may be employed in the present invention is a compound shown by the following Formula (V).

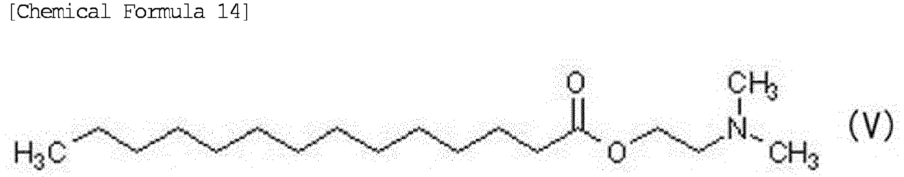

Akt Inhibitor VIII that may be employed in the present invention is a compound shown by the following Formula (VI).

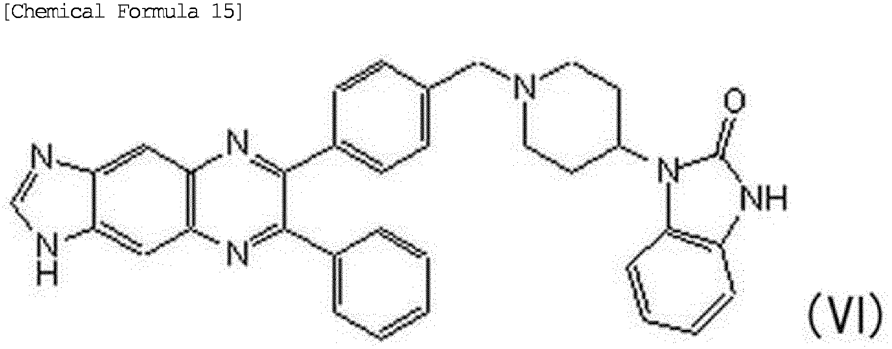

Although the present invention relates to the combination of ALAs and dynamin inhibitors, the aspects of the combination are not limited, and those skilled in the art can combine ALAs and dynamin inhibitors with various methods depending on the embodiments of PDT or PDD.

In the present invention, ALAs and dynamin inhibitors may be administered to a subject at same or different times. Moreover, in the present invention, ALAs and dynamin inhibitors may be prepared as formulations comprising each and then administered to a subject. Further, in the present invention, ALAs and dynamin inhibitors may be prepared as a kit that provides each separately.

In the present invention, an aspect for administering ALAs and dynamin inhibitors at the same time may be e.g. an aspect of administering to a subject a formulation comprising ALAs and dynamin inhibitors.

In the present invention, an aspect for administering ALAs and dynamin inhibitors at different times may be e.g. an aspect of administering ALAs and dynamin inhibitors each at different times, or may be an aspect of administering ALAs and dynamin inhibitors from different administration routes.

The administration route of ALAs and the administration route of dynamin inhibitors in the present invention are both not limited, and may be systemic administration or local administration. Administration routes include, for example, oral administration including sublingual administration, or parenteral administration such as inhalation administration, direct administration into the target tissue or organ via a catheter, intravenous administration including infusion, transdermal administration by e.g. patches, suppositories, or administration by forced enteral nutrition employing nasogastric tubes, nasointestinal tubes, and gastrostomy or enterostomy tubes. Moreover, as described above, ALAs and dynamin inhibitors may be administered from separate routes.

The dosage form of ALAs, dynamin inhibitors, or a formulation of a combination thereof used in the present invention may be appropriately determined depending on the said administration route, and can include, but is not limited to, for example injections, infusions, tablets, capsules, fine granules, powders, liquids, solutions dissolved in syrups etc., patches, and suppositories.

Although the present invention relates to the combination of ALAs and dynamin inhibitors, it can further use in combination a substance that increases the amount of PpIX accumulated in cells via a mechanism different from dynamin inhibitors. A substance that may increase the amount of PpIX accumulated in cells via a mechanism different from dynamin inhibitors includes for example ABCG2 inhibitors such as Fumitremorgin C, chelators, and activated form of vitamin D. ABCG2 inhibitors that may be optionally employed in the present invention include, for example, compounds described in Tables 3 and 4 of Mo et al., Int J Biochem Mol Biol 2012:3(1) :1-27, compounds described in Table 2 of Nakanishi et al., Chin J Cancer; 2012; Vol. 31 Issue 2, p75-99, and the like. It is described that chelators may be employed for increasing the amount of PpIX accumulated in cells in e.g. WO2014/2023833 or Valdes et al., Photochem Photobiol. 2010 Mar-Apr; 86 (2) : 471-5. Moreover, it is described that activated form of vitamin D may be employed for increasing the amount of PpIX accumulated in cells in e.g. Anand et al., Cancer Res; 71(18) September 15, 2011, 6040-6050.

Needless to say, other optional ingredients such as other medicinal ingredients, nutrients, and carriers can be added as necessary to the pharmaceutical according to the present invention. For example, as optional ingredients, various compounding ingredients for preparation of drugs such as pharmaceutically acceptable ordinary carriers, binders, stabilizers, solvents, dispersion mediums, expanders, excipients, diluents, pH buffers, disintegrants, solubilizers, solubilization aids, and isotonic agents, such as crystalline cellulose, gelatin, lactose, starch, magnesium stearate, talc, vegetable and animal fat, oil, gum, and polyalkylene glycol can be added.

In the present invention, the dosage of ALAs to a subject can be appropriately determined by those skilled in the art (such as a physician) depending on the embodiments of PDT or PDD. Without being limiting, administration can be 1 mg - 1,000 mg, preferably 5 mg - 100 mg, more preferably 10 mg - 30 mg, and further preferably 15 mg - 25 mg per kg of subject body weight, when converted into ALA.

The administration frequency of ALAs can be exemplified by administration at once to multiple times a day, or continuous administration such as by infusion.

The administration duration of ALAs can be determined by the pharmacologist or clinician skilled in the art with known methods for example in light of the symptom or condition of the subject to be prevented or treated etc. and based on various clinical indicators etc.

In the present invention, since dynamin inhibitor is administered with the purpose of potentiating the accumulation in the target tissue of PpIX produced by metabolism of ALAs, it is preferably administered together with the administration of ALAs, but does not necessarily need to be administered completely simultaneously.

The dynamin inhibitor in the present invention can be appropriately determined by those skilled in the art (such as a physician) depending on the embodiments of PDT or PDD and depending on the type of agents used.

ALA-PDT or ALA-PDD employing the present invention may be favorably employed for e.g. therapy or diagnosis of tumors and therapy or diagnosis of infections. When the present invention is employed for therapy or diagnosis of tumors, the type of the tumor is not particularly limited, and for example, targets for PDT can be favorably exemplified by superficial cancers such as warts, cervical cancer, skin cancer, thyroid cancer, and malignant brain tumor, or subcutaneous cancers, in particular cancers that are a few millimeters under the skin, and targets for PDD can be favorably exemplified by sentinel lymph nodes. Moreover, PDD allows diagnosis of lymph node metastasis before resection. When the present invention is employed for therapy or diagnosis of infections, the infection may be for example bacterial infection, fungal infection, viral infection, or parasitic infection, preferably it may be employed for bacterial infection, and further preferably it may be employed for staphylococcal infection or Pseudomonas aeruginosa infection.

The terms used herein, except for those that are particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples. However, the present invention can be embodied by various aspects, shall not be construed as being limited to the Examples described herein.

### Examples

### Example 1: Potentiation of PpIX accumulation in cancer cells by dynamin inhibitors

### Materials and Methods

Six types of cells strains were employed as cancer cell strains: bladder cancer cell strain (DU145), large intestine cancer cell strains (HT29, HCC2998), stomach cancer cell strain (MKN7), and mammary gland cancer cell strains (MDA-MB-231, MCF-7) . For the medium, RPMI 1640 (from Wako) supplemented with 5% inactivated fetal bovine serum (FBS, from Biowest) was employed (hereinafter referred to as RPMI medium) . Two types of inhibitors are known to exist for dynamin: one is an inhibitor that targets the GAS domain, whereas the other is an inhibitor that targets the PH domain. Iminodyn22 (from abcam) was used as the inhibitor that targets the GAS domain. Further, OcTMAB (from abcam) was used as the inhibitor that targets the PH domain.

To 1 mg of OcTMAB was added 255 µL of dimethyl sulfoxide (DMSO, from Wako), and this was mixed to make an OcTMAB 10 mM stock solution. Further, to 1 mg of Iminodyn22 was added 208 µL of DMSO, and this was mixed to make an Iminodyn22 10 mM stock solution. The OcTMAB 10 mM stock solution was diluted with the RPMI medium to adjust to 16 µM. The Iminodyn22 10 mM stock solution was diluted with the RPMI medium to adjust to 40 µM. The OcTMAB and Iminodyn22 prepared were each serially diluted with the RPMI medium to create six serial dilutions. Each of these dynamin inhibitor-containing medium was mixed with an equal amount of the RPMI medium comprising 2 mM aminolevulinic acid hydrochloride salt (ALA) to prepare ALA/OcTMAB and ALA/Iminodyn22-containing mediums.

Each cancer cell strain was seeded in a 96-well plate (black with transparent bottom; from Corning) at a density of 1.25×10⁴ cells/well (only MDA-MB-231 was seeded at 2.0×10⁴ cells/well), and this was cultured overnight at 37°C. After the overnight culture, the culture supernatant was removed, 200 µL each of ALA/OcTMAB-containing medium or ALA/Iminodyn22-containing medium described above were added to each well, and this was cultured for 4 hours under a 37°C condition. After 4 hours, 100 µL of each culture supernatant were transferred to another 96-well plate, and this was set as the extracellular PpIX measuring plate. The remaining culture supernatant was removed, each cell strain was washed once with phosphate buffered saline (PBS, from Wako), and then 100 µL per well of 1% dodecyl sodium sulfate (SDS, from Wako) aqueous solution was added to dissolve the cells, and this was set as the intracellular PpIX measuring plate. Protoporphyrin IX standard sample (PpIX, from Funakoshi Co., Ltd.) was dissolved in DMSO solution to create 1 mM PpIX stock solution. The 1 mM PpIX stock solution was diluted with each of 1% SDS solution and RPMI medium, and PpIX standard solutions with final concentrations of 0 - 1000 nM were prepared. The PpIX standard solutions prepared with 1% SDS were added to unused wells in the intracellular accumulation PpIX measuring plate, and the standard solutions prepared with the RPMI medium were added to unused portions of the extracellular export PpIX measuring plate at 100 µL each. The fluorescence intensity of the intracellular PpIX measuring plate and the extracellular PpIX measuring plate was measured with a microplate reader (infinite M200PRO; from TECAN) under the condition of excitation light at 405 nm and measurement wavelength at 635 nm. After measurement, a standard curve was created from the fluorescence intensity of the PpIX standard solutions, and the amount of PpIX in each well was calculated from the standard curve.

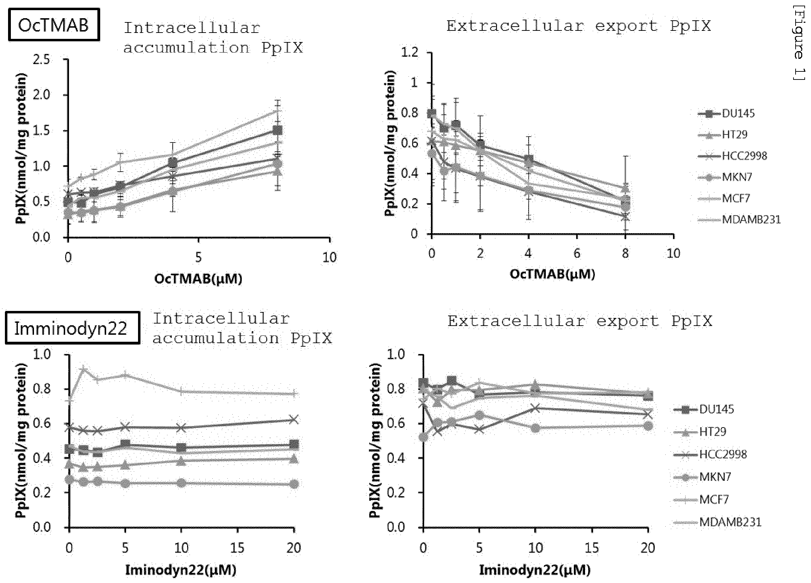

In order to correct the amount of PpIX measured by the above method with the amount of protein, the amount of protein contained in each sample of the intracellular PpIX measuring plate was measured. Pierce™ BCA Protein Assay Kit (from Thermo Fisher) was used for measuring the amount of protein. BCA reagent A and BCA reagent B were mixed at a proportion of 50:1 to prepare the BCA reaction solution. For the standard solutions for quantifying the amount of protein, the bovine serum albumin (BSA) solution attached to the kit was diluted to adjust to 0 - 2 mg/ml. To a 96-well plate, 25 µL each of the sample in the intracellular PpIX measuring plate was dispensed, and 200 µL of the BCA reaction solution were added to each. After reaction at 37°C for 30 minutes, absorbance at 562 nm was measured with a microplate reader (from TECAN) . After measurement, a standard curve was created from the absorbance of the BSA standard solutions, and the amount of protein for each well was calculated from the standard curve. The result of correcting the intracellular and extracellular PpIX amount with the amount of protein is shown in Figure 1.

### Results

As apparent from Figure 1, the use of ALA in combination with OcTMAB resulted in increase of intracellular PpIX amount dependent on the concentration of OcTMAB compared to when ALA was administered alone. Moreover, for the extracellular PpIX amount, the use of ALA in combination with OcTMAB resulted in decrease of extracellular PpIX amount dependent on the concentration of OcTMAB compared to when ALA was administered alone. In contrast, when ALA and iminodyn22 were used in combination, no change in the intracellular and extracellular PpIX amounts was confirmed. From the above results, it was suggested that by using an inhibitor that targets the PH domain of dynamin in combination with ALA, extracellular export of PpIX is inhibited and the amount of intracellular accumulation increases.

### Example 2: Potentiation of PpIX accumulation by combination use of OcTMAB and FTC

### Materials and Methods

Six types of cells strains were employed as cancer cell strains: bladder cancer cell strain (DU145), large intestine cancer cell strains (HT29, HCC2998), stomach cancer cell strain (MKN7), and mammary gland cancer cell strains (MDA-MB-231, MCF-7). Fumitremorgin C (FTC, from sigma) was dissolved in DMSO to create a 2 mM stock solution, and then adjusted to 40 µM with the RPMI medium. For OcTMAB, the 10 mM stock solution was adjusted with the RPMI medium to 32 µM. The 40 µM FTC-containing RPMI medium was added to an equal amount of 32 µM OcTMAB-containing medium to prepare a 16 µM OcTMAB/20 µM FTC-containing medium. Note that FTC is an inhibitor of ABCG2 (a transporter that extracellularly exports PpIX), and is known as a compound that has the effect of inhibiting extracellular export of PpIX.

Each cancer cell strain was seeded in a 96-well plate (black with transparent bottom) at a density of 1.25×10⁴ cells/well (only MDA-MB-231 was seeded at 2.0×10⁴ cells/well), and this was cultured overnight at 37°C. After the overnight culture, the culture supernatant was removed, 100 µL each of 2 mM ALA-containing RPMI medium was added to each well. Then, 100 µL each of 16 µM OcTMAB/20 µM FTC-containing medium described above was added, and this was cultured for 4 hours at 37°C (final concentration ALA 1 mM, OcTMAB 8 µM, FTC 10 µM). As comparison, effect of ALA alone group, ALA/FTC combination use group, and ALA/OcTMAB combination use group were also created at the same time. After 4 hours, 100 µL each of the culture supernatant in each well were transferred to another 96-well plate (black with transparent bottom), and this was set as the extracellular PpIX amount measuring plate. The remaining culture supernatant was removed, washed once with PBS, and then 100 µL per well of 1% SDS aqueous solution was added to dissolve the cells, and this was set as the intracellular PpIX amount measuring plate. The preparation of PpIX standard solutions and the intracellular and extracellular PpIX amount measuring method were carried out similarly to Example 1.

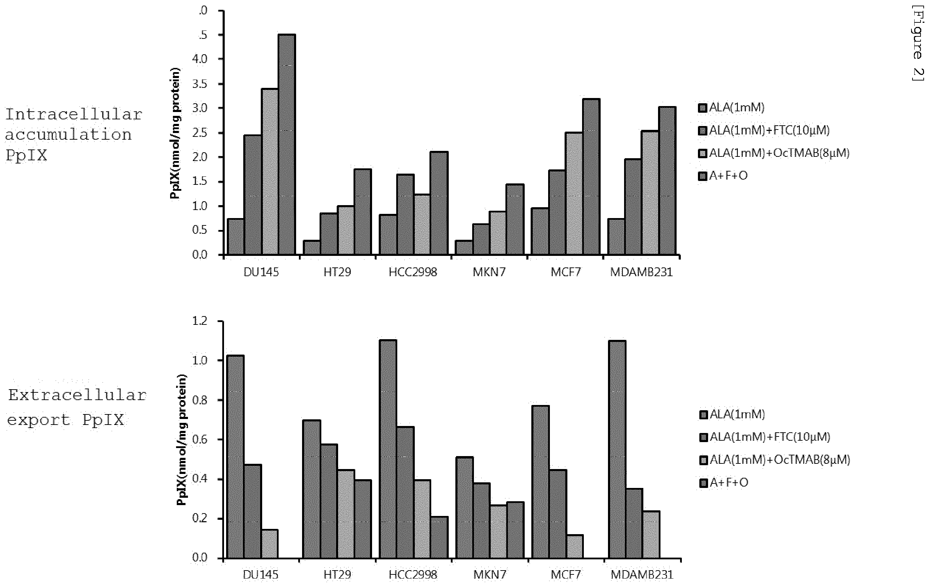

In order to correct the above PpIX amount measurement result with the amount of protein, the amount of protein in each sample for measuring the intracellular PpIX amount was measured. Measurement of the amount of protein carried out using Pierce™ BCA Protein Assay Kit (from Thermo Fisher) with a method similar to Example 1. The result of correcting the intracellular and extracellular PpIX amount with the amount of protein is shown in Figure 2.

### Results

As apparent from Figure 2, in many cell strains, the ALA and OcTMAB combination use group increased intracellular PpIX similar to or more than the ALA and FTC combination use group. Further, it was shown that the use of OcTMAB in combination with FTC tended to increase the intracellular PpIX amount compared to when each inhibitor was administered alone.

From the measurement result of extracellular PpIX amount, it was shown that OcTMAB had an effect similar to FTC for suppressing PpIX extracellular export. Moreover, it was found that the use of OcTMAB in combination with FTC decreases the extracellular PpIX amount compared to when OcTMAB or FTC was administered alone. From these results, it was suggested that OcTMAB was possibly acting on an export mechanism different from ABCG2.

### Example 3: Potentiation of PpIX accumulation in cancer cells by MiTMAB

### Materials and Methods

Six types of cells strains were employed as cancer cell strains: bladder cancer cell strain (DU145), large intestine cancer cell strains (HT29, HCC2998), stomach cancer cell strain (MKN7), and mammary gland cancer cell strains (MDA-MB-231, MCF-7). The RPMI medium was employed as the medium. Moreover, MiTMAB (from abcam) was employed as the dynamin inhibitor.

To 1 mg of MiTMAB was added 297 µL of DMSO, and this was mixed to make a 10 mM stock solution. The 10 mM stock solution was diluted with the RPMI medium to adjust to 32 µM. The MiTMAB-containing medium prepared was serially diluted with the RPMI medium to create six serial dilutions. Each concentration of the MiTMAB-containing medium was mixed with an equal amount of the RPMI medium comprising 2 mM ALA to prepare an ALA/MiTMAB-containing medium.

Each cancer cell strain was seeded in a 96-well plate at a density of 1.25×10⁴ cells/well (only MDA-MB-231 was seeded at 2.0×10⁴ cells/well), and this was cultured overnight at 37°C. After the overnight culture, the culture supernatant was removed, 200 µL of each ALA/MiTMAB-containing medium described above were added to each well, and this was cultured for 4 hours under a 37°C condition. After 4 hours, 100 µL of each culture supernatant were transferred to another 96-well plate, and this was set as the extracellular PpIX amount measuring plate. The remaining culture supernatant was removed and each of the cells was washed once with PBS, and then 100 µL per well of 1% SDS aqueous solution was added to allow dissolution, and this was set as the intracellular PpIX amount measuring plate. The preparation of PpIX standard solutions and the intracellular and extracellular PpIX amount measuring method were carried out similarly to Example 1.

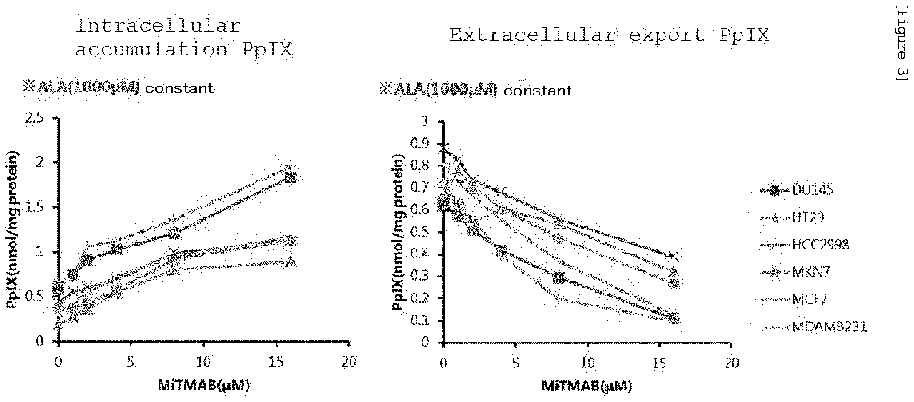

In order to correct the above result with the amount of protein, the amount of protein was measured using each sample in the intracellular PpIX measuring plate. Pierce™ BCA Protein Assay Kit (from Thermo Fisher) was used for measuring the amount of protein. The method for measuring the amount of protein was carried out similarly to Example 1. The result of correcting the intracellular and extracellular PpIX amount with the amount of protein is shown in Figure 3.

### Results

As apparent from Figure 3, the use of ALA in combination with MiTMAB resulted in increase of intracellular PpIX amount dependent on the concentration of MiTMAB compared to when ALA was administered alone. Moreover, for the extracellular PpIX amount, compared to when ALA was administered alone, the use of ALA in combination with MiTMAB resulted in decrease of extracellular PpIX amount dependent on the concentration of MiTMAB. From the above results, it was suggested that by using MiTMAB in combination, extracellular export of PpIX is inhibited similarly to OcTMAB in Example 1, and the amount of intracellular PpIX accumulation increases.

### Example 4: Potentiation of PpIX accumulation in cancer cells by RTIL13

### Method

Six types of cells strains were employed as cancer cell strains: bladder cancer cell strain (DU145), large intestine cancer cell strains (HT29, HCC2998), stomach cancer cell strain (MKN7), and mammary gland cancer cell strains (MDA-MB-231, MCF-7) . The RPMI medium was employed as the medium. Moreover, RTIL13 (from abcam) was employed as the dynamin inhibitor.

To 5 mg of RTIL13 was added 875 µL of DMSO, and this was mixed to make a 10 mM stock solution. The 10 mM stock solution was diluted with the RPMI medium to adjust to 40 µM. The RTIL13-containing medium prepared was serially diluted with the RPMI medium to create six serial dilutions. Each concentration of the RTIL13-containing medium was mixed with an equal amount of the RPMI medium comprising 2 mM ALA to prepare an ALA/RTIL13-containing medium.

Each cancer cell strain was seeded in a 96-well plate at a density of 1.25×10⁴ cells/well (only MDA-MB-231 was seeded at 2.0×10⁴ cells/well), and this was cultured overnight at 37°C. After the overnight culture, the culture supernatant was removed, 200 µL of each ALA/RTIL13-containing medium described above were added to each well, and this was cultured for 4 hours under a 37°C condition. After 4 hours, 100 µL of each culture supernatant were transferred to another 96-well plate, and this was set as the extracellular PpIX amount measuring plate. The remaining culture supernatant was removed and each of the cells was washed once with PBS, and then 100 µL per well of 1% SDS aqueous solution was added to allow dissolution, and this was set as the intracellular PpIX amount measuring plate. The preparation of PpIX standard solutions and the intracellular and extracellular PpIX amount measuring method were carried out similarly to Example 1.

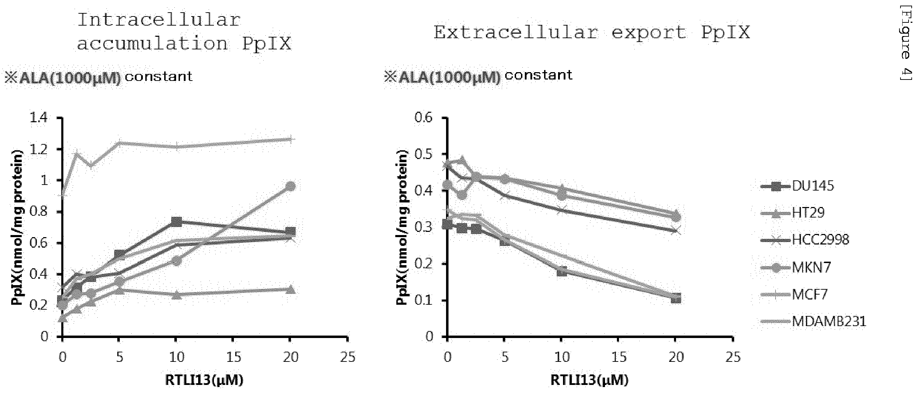

In order to correct the above result with the amount of protein, the amount of protein was measured using each sample in the intracellular PpIX measuring plate. Pierce™ BCA Protein Assay Kit (from Thermo Fisher) was used for measuring the amount of protein. The method for measuring the amount of protein was carried out similarly to Example 1. The result of correcting the intracellular and extracellular PpIX amount with the amount of protein is shown in Figure 4.

### Results

As apparent from Figure 4, the use of ALA in combination with RTIL13 resulted in increase in the amount of PpIX accumulated peaking at around RTIL13 concentration at 10 µM, compared to when ALA was administered alone. Moreover, for the extracellular PpIX amount, compared to when ALA was administered alone, the use of ALA in combination with RTIL13 resulted in decrease of extracellular PpIX amount dependent on the concentration of RTIL13. From this result, it was suggested that by using RTIL13 in combination, extracellular export of PpIX is inhibited similarly to the results of Example 1 and 3, and the amount of intracellular PpIX accumulation increases.

OcTMAB, MiTMAB, and RTIL13 are all inhibitors that target the PH domain of dynamin. From the results of Examples 1 - 4, it was suggested that by using an inhibitor that targets the PH domain of dynamin in combination with ALA, the amount of PpIX accumulated in cells can be increased.

### Example 5: Potentiation of PpIX accumulation in cancer cells by AKT inhibitor VIII

Next, an attempt was made to increase the amount of PpIX accumulated in cells by an agent that is not generally known as a dynamin inhibitor but binds to the PH domain structure and inhibits protein activity.

### Method

Six types of cells strains were employed as cancer cell strains: bladder cancer cell strain (DU145), large intestine cancer cell strains (HT29, HCC2998), stomach cancer cell strain (MKN7), and mammary gland cancer cell strains (MDA-MB-231, MCF-7) . The RPMI medium was employed as the medium. As the test agent, Akt inhibitor VIII (from Merck Millipore) that is known to bind to the PH domain of Akt and inhibits the activity of Akt was employed.

To 1 mg of Akt inhibitor VIII was added 363 µL of DMSO, and this was mixed to make a 5 mM stock solution. 5 mM stock solution was diluted with the RPMI medium to adjust to 40 µM. The 40 µM Akt inhibitor VIII-containing medium prepared was serially diluted with the RPMI medium to create six serial dilutions. Each concentration of the AKT inhibitor VIII-containing medium was mixed with an equal amount of the RPMI medium comprising 2 mM ALA to prepare an ALA/Akt inhibitor VIII-containing medium.

Each cancer cell strain was seeded in a 96-well plate at a density of 1.25×10⁴ cells/well (only MDA-MB-231 was seeded at 2. 0×10⁴ cells/well), and this was cultured overnight at 37°C. After the overnight culture, the culture supernatant was removed, 200 µL of each ALA/Akt inhibitor VIII-containing medium described above were added to each well, and this was cultured for 4 hours under a 37°C condition. After 4 hours, 100 µL of each culture supernatant were transferred to another 96-well plate, and this was set as the extracellular PpIX amount measuring plate. The remaining culture supernatant was removed and each of the cells was washed once with PBS, and then 100 µL per well of 1% SDS aqueous solution was added to allow dissolution, and this was set as the intracellular PpIX amount measuring plate. The preparation of PpIX standard solutions and the intracellular and extracellular PpIX amount measuring method were carried out similarly to Example 1.

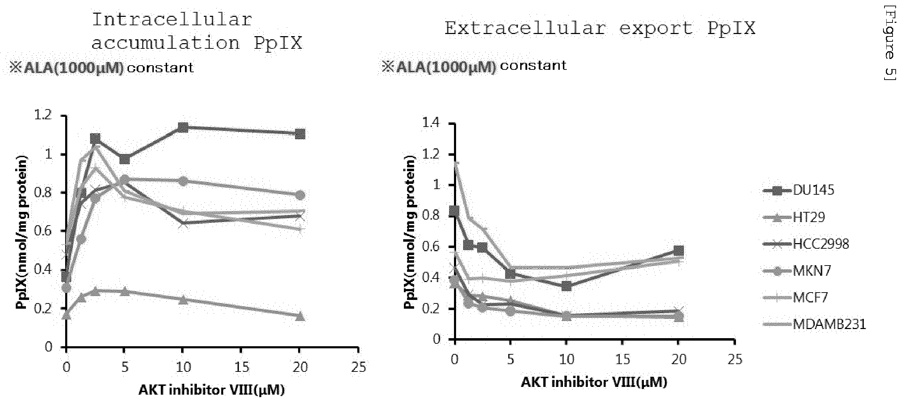

In order to correct the above result with the amount of protein, the amount of protein was measured using each sample in the intracellular PpIX measuring plate. Pierce™ BCA Protein Assay Kit (from Thermo Fisher) was used for measuring the amount of protein. The method for measuring the amount of protein was carried out similarly to Example 1. The result of correcting the intracellular and extracellular PpIX amount with the amount of protein is shown in Figure 5.

### Results

As apparent from Figure 5, the use of ALA in combination with Akt inhibitor VIII resulted in increase in the amount of PpIX accumulated peaking at around Akt inhibitor VIII concentration at 5 µM, compared to when ALA was administered alone. Moreover, for the extracellular PpIX amount, compared to when ALA was administered alone, the use of ALA in combination with Akt inhibitor VIII resulted in decrease of extracellular PpIX amount peaking at around Akt inhibitor VIII concentration at 5 µM. From this result, it was suggested that by using Akt inhibitor VIII in combination, extracellular export of PpIX is inhibited similarly to the result of Examples 1, 3, and 4, and the amount of intracellular PpIX accumulation increases.

From the above results, it was suggested that even an agent that is not generally known as a dynamin inhibitor may be used as a "dynamin inhibitor" of the present invention if it is an agent that binds to the PH domain structure and inhibits protein activity.

## Claims

1. A pharmaceutical for potentiating photodynamic reaction in photodynamic therapy or photodynamic diagnosis, **characterized in that** it comprises:
(A) a compound shown by the following Formula (I): 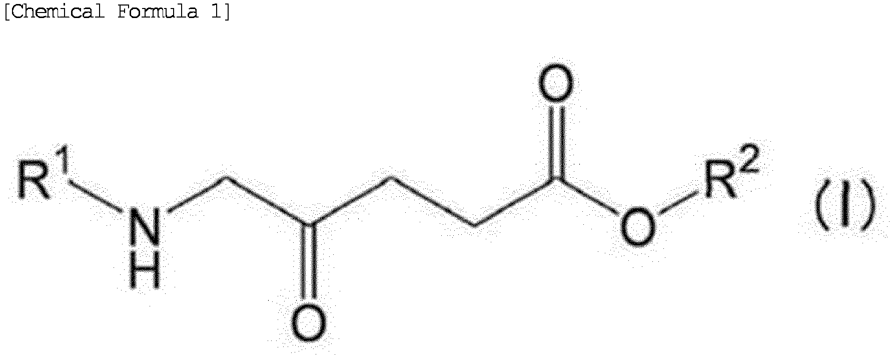
(in which R¹ represents a hydrogen atom or an acyl group, and R² represents a hydrogen atom, a linear or branched alkyl group, a cycloalkyl group, an aryl group, or an aralkyl group)
or a salt thereof, and
(B) a dynamin inhibitor.

2. A combination pharmaceutical which is administered at same or different times for potentiating photodynamic reaction in photodynamic therapy or photodynamic diagnosis, which is:
(A) a compound shown by the following Formula (I): 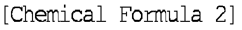 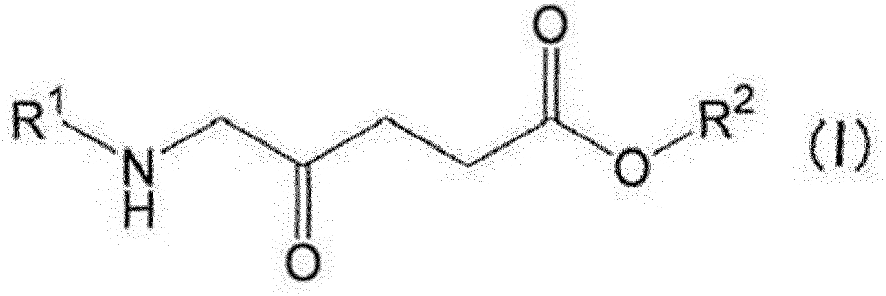
(in which R¹ represents a hydrogen atom or an acyl group, and R² represents a hydrogen atom, a linear or branched alkyl group, a cycloalkyl group, an aryl group, or an aralkyl group)
or a salt thereof, and
(B) a dynamin inhibitor.

3. A pharmaceutical for potentiating photodynamic reaction in photodynamic therapy or photodynamic diagnosis **characterized in that** said pharmaceutical comprises a compound shown by the following Formula (I): 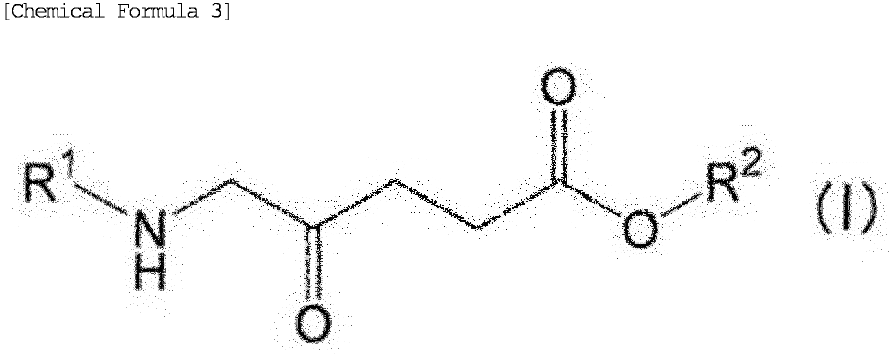
(in which R¹ represents a hydrogen atom or an acyl group, and R² represents a hydrogen atom, a linear or branched alkyl group, a cycloalkyl group, an aryl group, or an aralkyl group)
or a salt thereof, and
said pharmaceutical is used in combination with a dynamin inhibitor.

4. The pharmaceutical according to claims 1 to 3, **characterized in that**:
R¹ is selected from the group consisting of a hydrogen atom, an alkanoyl group having 1 - 8 carbons, and an aroyl group having 7 - 14 carbons, and
R² is selected from the group consisting of a hydrogen atom, a linear or branched alkyl group having 1 - 8 carbons, a cycloalkyl group having 3 - 8 carbons, an aryl group having 6 - 14 carbons, and an aralkyl group having 7 - 15 carbons.

5. The pharmaceutical according to claim 4, **characterized in that** R¹ and R² are hydrogen atoms.

6. The pharmaceutical according to any one of claims 1 to 5, **characterized in that** said dynamin inhibitor is a dynamin inhibitor that targets the pleckstrin homology domain of dynamin.

7. The pharmaceutical according to claim 6, **characterized in that** said dynamin inhibitor that targets the pleckstrin homology domain of dynamin is one or more compound selected from the group consisting of the following Formula (II) - Formula (VI).

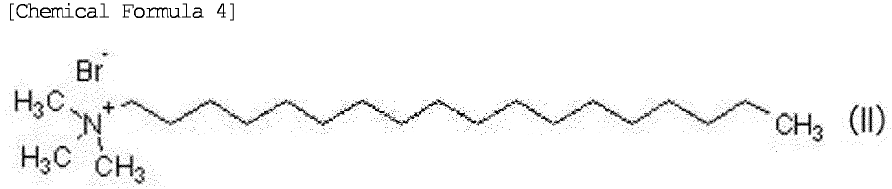

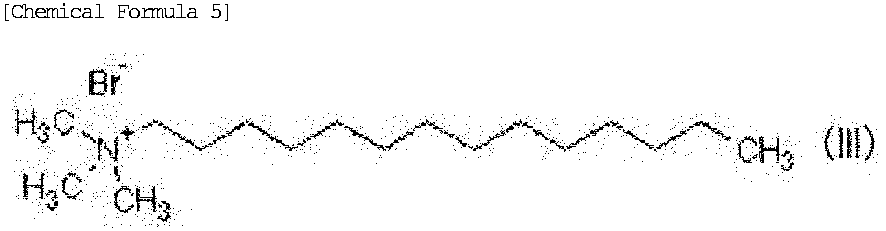

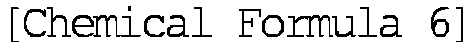

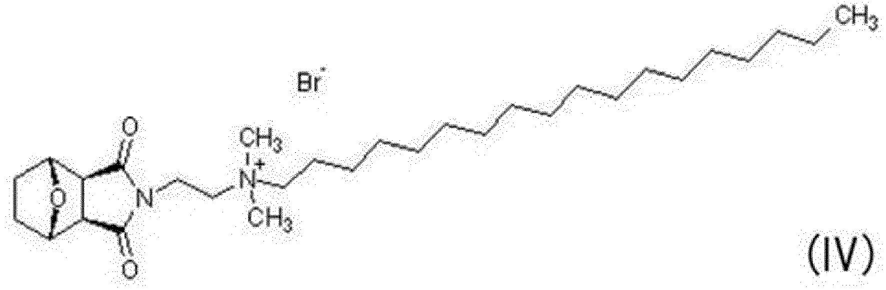

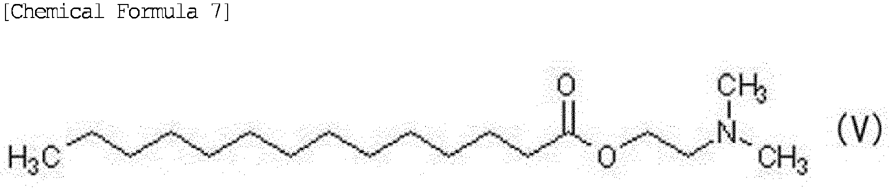

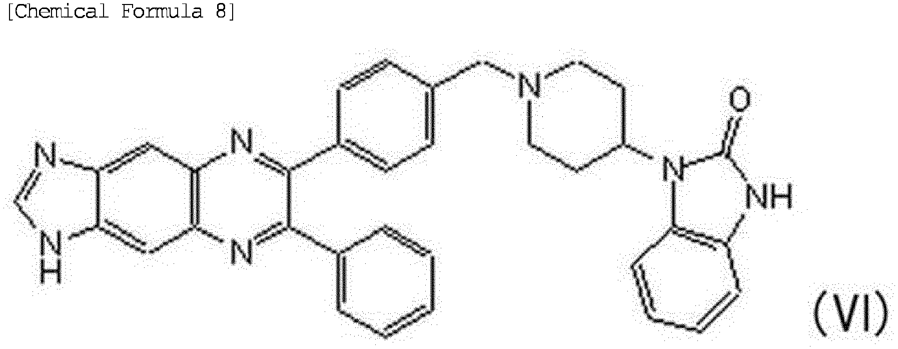

8. The combination pharmaceutical according to claim 2, **characterized in that** said (A) and said (B) are prepared as a kit.

9. A method for potentiating photodynamic reaction in photodynamic therapy or photodynamic diagnosis that employs a compound shown by the following Formula (I): 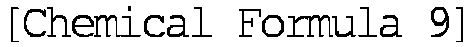 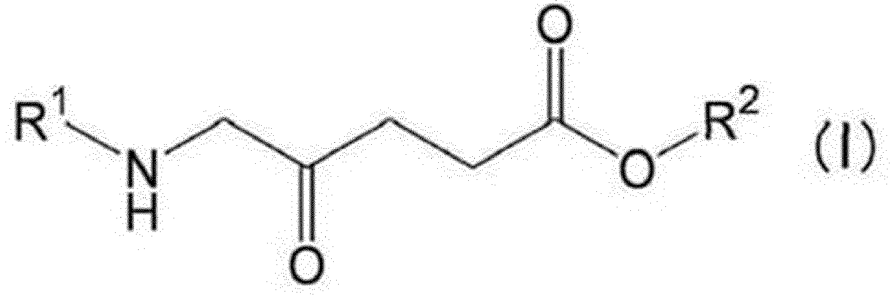
(in which R¹ represents a hydrogen atom or an acyl group, and R² represents a hydrogen atom, a linear or branched alkyl group, a cycloalkyl group, an aryl group, or an aralkyl group)
or a salt thereof, **characterized in that** it comprises:
a step of administering to a subject a therapeutically effective amount of a dynamin inhibitor.
